# EUROPÄISCHE PATENTSCHRIFT

(11) **EP 2 713 975 B1**
(45) Veröffentlichungstag und Bekanntmachung des Hinweises auf die Patenterteilung: **30.12.2015**
(21) Anmeldenummer: 12730147.1
(22) Anmeldetag: 30.05.2012
(51) Int. Cl.: A61F 13/551, B65D 85/16, B65B 9/067

(54) **VERPACKUNGSANLAGE ZUM VERPACKEN VON FLACHEN, BIEGSAMEN PRODUKTEN**
PACKING LINE FOR PACKING FLAT, FLEXIBLE PRODUCTS
LIGNE D'EMBALLAGE POUR EMBALLER DES PRODUITS PLATS ET FLEXIBLES

(30) Priorität: 30.05.2011 DE 102011103560
(43) Veröffentlichungstag der Anmeldung: 09.04.2014
(73) Patentinhaber: Winkler + Dünnebier GmbH, 56564 Neuwied (DE)
(72) Erfinder: RINKE, Andreas, 23843 Bad Oldesloe (DE); MAJEWSKI, Rolf, 53579 Erpel (DE); SEGER, Reiner, 56566 Neuwied (DE); VIEWEG, Jürgen, 56220 Sankt Sebastian (DE); BRETZ, Michael, 56567 Neuwied (DE); LAMPRECHT, Ulrich, 56567 Neuwied (DE); LUGOJA, Lucian-Gabriel, 67596 Dittelsheim-Hessloch (DE)
(74) Vertreter: Walkenhorst, Andreas
(86) Internationale Anmeldenummer: PCT/EP2012/002284
(87) Internationale Veröffentlichungsnummer: WO 2012/163522

(56) Entgegenhaltungen:
- US-A- 5 564 261

## Beschreibung

Die Erfindung betrifft eine Verpackungsanlage zum Verpacken von flachen, biegsamen Produkten, insbesondere von Hygieneprodukten, in Verpackungsbeutel, mit einer um einen Formschuh kontinuierlich laufenden Schlauchfolie zum Umhüllen der Produkte mit dem gebildeten Folienschlauch, einer Fördereinrichtung zum Zu- und Einführen der Produktstapel in den Folienschlauch sowie nachfolgenden Schweißeinrichtungen für die Quer- und Längsschweißung des Folienschlauchs.

Hygieneprodukte wie beispielsweise Papiertaschentücher, Slipeinlagen, Damenbinden, Windeln oder dergleichen werden üblicherweise einzeln oder in vorgegebenen Chargen, beispielsweise in der Art von Fünfer- oder Zehnerpacks; in Folie verpackt und in dieser verpackten Form verbrauchs- oder gebrauchsfertig angeboten. Die das Hygieneprodukt bzw. den Stapel der Hygieneprodukte umgebende Folie bildet dabei einen so genannten Verpackungsbeutel, der u.a. beispielsweise mit einer Perforation zum erleichterten Öffnen und/oder mit Klebelaschen oder dergleichen zum Wiederverschließen ausgerüstet sein kann. Gleichermaßen können auch andere Produkte oder Produktchargen, wie beispielsweise Zigarettenpackungen oder dergleichen, mit einem derartigen Verpackungsbeutel in der Art einer Umverpackung versehen werden.

Zum Einbringen des Produkts, insbesondere des Hygieneprodukts oder des Stapels von Hygieneprodukten, in den Verpackungsbeutel kann es beispielsweise vorgesehen sein, dass geeignet zugeschnittene, zur Bildung des Verpackungsbeutels vorgesehene Folienstücke bereitgestellt werden, in die das Produkt eingelegt wird, wobei die Folie anschließend um dieses herumgeschlagen und an den Rändern verschweißt wird. Ein derartiges Konzept zum Verpacken von Hygieneprodukten in einen Verpackungsbeutel ist beispielsweise aus der DE 101 48 283 A1 bekannt, bei der ein so genanntes Zellenrad für den eigentlichen Verpackungsschritt vorgesehen ist. Dabei wird die zur Bildung des Verpackungsbeutels vorgesehene Folie zunächst in eine Kammer des Zellenrades eingelegt, wobei sodann ein Stapel von Hygieneprodukten ebenfalls in die Kammer des Zellenrades eingebracht wird. Anschließend werden die Seitenränder der Folie umlaufend um den Stapel von Hygieneprodukten herum in Überlappung gebracht und miteinander geeignet verschweißt.

Alternativ kann auch ein so genannter Folienschlauch-Verpacker zum Einsatz kommen, wie er beispielsweise aus der DE 10 2008 020 800 A1 bekannt ist. In einem derartigen System werden die zu verpackenden Hygieneprodukte bzw. Stapel von Hygieneprodukten in einer so genannten Schlauchbeutelmaschine in einen Folienschlauch positioniert, der dann zwischen den Produkten zusammengeschweißt und getrennt wird.

Die US 5,564,261 beschreibt ein Verfahren und eine Vorrichtung, bei der komprimierte Artikel einer Umhüllungsmaschine zugeführt werden. Dabei werden die zusammengepressten Gegenstände über ein Förderband bis zu einer Endposition transportiert und dann mit einer Folie mehrfach röhrenförmig umhüllt, so dass gewährleistet ist, dass die Folie die Artikel in dem komprimierten Zustand zuverlässig zusammenhält.

Gerade beim Verpacken von Hygieneprodukten, die in besonders großer Stückzahl hergestellt werden, wie beispielsweise Papiertaschentücher, Slipeinlagen, Damenbinden oder dergleichen, ist eine hohe Bearbeitungsgeschwindigkeit und Durchsatzrate beim Verpacken der Produkte wünschenswert. Aus diesem Grund werden die Verpackungssysteme üblicherweise in der Art von Durchlaufsystemen ausgelegt. Bei diesen werden die zu verpackenden Produkte aufeinander folgend, einzeln oder stapelweise entlang einer Transportrichtung weiterbefördert und während des Durchlaufs durch verschiedene, entlang der Transportrichtung angeordnete Systemkomponenten mit dem Verpackungsbeutel umgeben. Insbesondere die genannten Folienschlauchsysteme sind für hohe Bearbeitungsgeschwindigkeiten und Durchsatzraten grundsätzlich gut geeignet, da beispielsweise beim Einschlagen der Produkte oder Produktstapel in die Schlauchfolie zur Bildung des Folienschlauchs ein Verschweißen der seitlichen Längsnaht von der Seite her, also im Durchlauf und ohne Systemstillstand, möglich ist. Um diesen Vorteil gerade bei Folienschlauchsystemen auch im Gesamtprozess nutzbar zu machen und aus Gründen des möglichst geringem Materialverbrauchs sowie aus optischen Gründen besteht das Bestreben die Produktstapel eng mit der Schlauchfolie zu umschließen, so dass diese möglichst keine Bewegungen innerhalb des Verpackungsbeutels ausführen können. Die sich im Durchlauf befindenden Produktstapel müssen während des kontinuierlichen Transports mit der Schlauchfolie umhüllt werden. Dabei soll die Schlauchfolie an allen Seiten straff und glatt den Produktstapel umhüllen und an keiner Seite bzw. Fläche Falten aufweisen.

Der vorliegenden Erfindung liegt daher die Aufgabe zugrunde, eine Möglichkeit anzugeben, bei einem kontinuierlichen Transport flache, biegsame Produktstapel reib- und formschlüssig festzuhalten um sie eng und formschlüssig mit einer Verpackungsfolie zu umhüllen.

Diese Aufgabe wird erfindungsgemäß gelöst mit einer Fördereinrichtung, die als Bandförderer mit seitlich an den Produkten angreifenden Transportbändern ausgeführt ist, wobei die Transportbänder aus mindestens zwei Einzelbänder bestehen und wobei die Einzelbänder an mindestens einer Position innerhalb der Vepackungsanlage überlappend gelagert sind.

Die Erfindung geht dabei von der Überlegung aus, dass zur Erreichung besonders hoher Durchsatz- und Produktionsraten ein möglichst kontinuierlicher und unterbrechungsfreier horizontaler Durchlauf der Produkte oder Produktstapel durch das Verpackungssystem angestrebt werden sollte. Um dabei Systemstillstände oder auch kurzfristige Unterbrechungen des Weitertransports der Produkte oder Produkt Stapel weitestgehend zu verhindern, sollten sämtliche Bearbeitungsschritte beim Einbringen der Produkte oder Produktstapel in die jeweiligen Verpackungsbeutel konsequent für einen Durchlaufbetrieb ausgelegt sein. Die herkömmlichen Methoden zum Umhüllen der Produktstapel mit der Schlauchfolie, die hauptsächlich durch den Einsatz von Zellenrädern erfolgt, sind hierfür aber gerade nicht geeignet. Aufgrund der hohen Durchlaufgeschwindigkeit derartiger Verpackungssysteme, muss gewährleistet sein, wenn die umhüllten Produkte oder Produktstapel frei von Mängeln herauskommen sollen, diese entlang des gesamten Umhüllungsvorgangs und den danach folgenden Abläufen, wie beispielsweise, schließen der offenen Seiten der Schlauchfolie durch Schweißen, hindurch kontinuierlich und voll unter Kontrolle zu halten, so dass jede Möglichkeit des Verschiebens zwischen den Produkten oder der Produktstapel und der Schlauchfolie vermieden wird, was zu unansehnlichen Falten oder Mängeln führen könnte.

Um dem entgegenzuwirken, sollte beim Zuführen der zu verpackenden flachen, biegsamen, Produkte, insbesondere der Produktstapel, zum Umhüllen mit einem Folienschlauch, die Fördereinrichtung vom Grundsatz her auf einem andersartigen Konzept aufgebaut sein. Dazu ist nunmehr vorgesehen, dass die Fördereinrichtung als Bandförderer mit seitlich an den Produkten angreifenden Transportbändern ausgeführt ist. Dabei bestehen die Transportbänder aus mindestens zwei Einzelbändern, die an mindestens einer Position innerhalb der Verpackungsanlage überlappend gelagert ausgeführt sind. Diese vorteilhafte Ausgestaltung bedingt, dass die Produkte beim Zu- und Einführen in den, über einen Formschuh gebildeten, Folienschlauch, sowie auch beim Austragen aus dem Formschuh im mit Folie umhüllten Zustand, immer durch mindestens eins der Transportbänder seitlich festgehalten geführt werden. Durch diesen mechanischen, reibschlüssigen Zugriff im Zuführ- sowohl als auch im Austragsbereich, wird ein Verschieben des Produkts vermieden. Vorzugsweise werden die Produkte vor, während und nach der Umhüllung mit der Schlauchfolie, derart von den Einzelbändern geführt, dass diese sich gegenseitig abstützen und die Produkte bzw. der Folienschlauch dazwischenliegend geklemmt geführt wird.

Um die zu fördernden Produkte und den Folienschlauch zuverlässig zu führen, können die übereinander positionierten Einzelbänder die gleiche oder aber auch eine unterschiedliche Breite aufweisen.

Vorteilhafterweise kann der Abstand der parallel geführten Einzelbänder variabel positioniert sein, so dass sie voneinander beabstandet oder zueinander angrenzend geführt sind.

Durch die vorteilhafte Ausgestaltung, dass zum ständigen reibschlüssigen seitlichen Zugriff immer jeweils mindestens ein Einzelband im Austragsbereich bis in den nächsten Zuführbereich hineinreicht und auch durch die Positionierung und der Breite der Einzelbänder ist ein mechanischer, reibschlüssiger Zugriff auf die Produkte und den Folienschlauch und somit eine geklemmte Führung während des gesamten Transports gewährleistet. Für diese geklemmte und lückenlose Führung des Produktstapels durch den Formschuh, in dem dann auch die Umhüllung mit der Schlauchfolie erfolgt, durch eine Art Übergabestation innerhalb des Formschuhs. Im Bereich der Übergabestation sind die Einzelbänder überlappend gelagert. Um den seitliche Zugriff auf die Produktstapel positiver zu unterstützen können die Einzelbänder mit geeignet gewählter, beispielsweise gummierter Oberfläche ausgebildet sein.

Vorteilhafterweise sind die Einzelbänder des Transportsystems, insbesondere im einem Zuführ- und Austragsbereich des Formschuhs und im Bereich der Übergabestation über Umlenkrollen geführt. Um dabei einen besonders störungsfreien Verfahrensablauf zu ermöglichen, sind die Umlenkrollen in besonders vorteilhafter Ausgestaltung in ihrer Dimensionierung und/oder hinsichtlich des Abstandes ihrer Drehachsen voneinander, insbesondere in Transportrichtung der Produkte gesehen, versetzt gelagert. In weiterer Ausgestaltung können die Umlenkrollen der überlappend gelagerten Einzelbänder dieselbe Drehachse aufweisen. Gerade bei den überlappend gelagerten Einzelbändern ist die Position der Umlenkrollen variierbar. Sie können dieselbe Drehachse aufweisen, können aber auch hinsichtlich ihrer Drehachsen beabstandet sein. Wichtig ist nur, dass die Einzelbänder an mindestens einer Position innerhalb der Verpackungsanlage, vorzugsweise innehalb des Formschuhs im bereich der Übergabestation, überlappend gelagert sind Vorzugsweise ist dabei dem Auslegungsziel eines zuverlässigen Zugriffs auf die Produkte und den Folienschlauch bevorzugt von der Seite, durch die Einzelbänder dadurch Rechnung getragen, dass einerseits die Umlenkrollen derart positioniert sind, dass die Einzelbänder an den, in Form einer Dreiecksspitze anlaufenden Zuführbereich des Formschuhs nahezu heranreichen. Dazu ist der Abstand der Drehachsen der Umlenkrolle im Zuführbereich versetzt gelagert. Zudem ist eines der im Zuführbereich vorgesehenen verhältnismässig schmalen Einzelbänder im unteren Drittel, sozusagen in einem Fußbereich, des Fördersystems angeordnet, während das parallel darüber beabstandet angeordnete Einzelband im oberen Drittel, sozusagen im Kopfbereich, verläuft. Andererseits wird das Produkt mit unmittelbarem Beginn der Umhüllung mit Schlauchfolie schon innerhalb des Formschuhs von einem nächsten Einzelband, im Bereich der Übergabestation, gegriffen. Dies ist nur dadurch möglich, dass das im Zuführbereich im oberen Drittel verlaufende Einzelband und das im Bereich der Übergabestation im unteren Drittel angeordnete Einzelband überlappend gelagert sind. Dabei können die Umlenkrollen dieser beiden Einzelbänder dieselbe Drehachse aufweisen und an einer einzelnen Umlenkrolle entsprechend beabstandet gemeinsam geführt werden oder aber es sind zwei Umlenkrollen vorgesehen, die entsprechend übereinander positioniert sind und somit dieselbe Drehachse aufweisen, oder aber die Drehachsen der Umlenkrollen der überlappend gelagerten Einzelbänder sind versetzt gelagert.

Vorteilhafterweise ist der Formschuh in geteilter Form auf einer Linearführung ausgebildet. Durch diese vorteilhafte Ausgestaltung können unterschiedliche Produkte mit unterschiedlicher Dicke, beispielsweise Fünfer- oder Zehnerpacks eines Hygieneprodukts, zuverlässig durch den Formschuh transportiert und mit Schlauchfolie umhüllt werden.

Ein zusätzlich am Formschuh vorgesehener Niederhalter gewährleistet, dass im Ausführungsbereich die Längsseiten des Folienschlauchs bis zur folgenden Schweißeinrichtung zur Längsschweißung des Folienschlauchs zuverlässig überlappend liegend weitergefördert werden.

Ein Ausführungsbeispiel der Erfindung wird anhand einer Zeichnung näher erläutert. Darin zeigen:
- FIG. 1: eine Verpackungsanlage zum Verpacken von Hygieneprodukten,
- FIG. 2: die Verpackuhgsanlage nach FIG. 1 im Ausschnitt mit einem Formschuh (Draufsicht),
- Fig. 3: die Verpackungsanlage nach FIG. 1 im Ausschnitt mit einem Formschuh (Schnitt).

Gleiche Teile sind in allen Figuren mit denselben Bezugszeichen versehen.

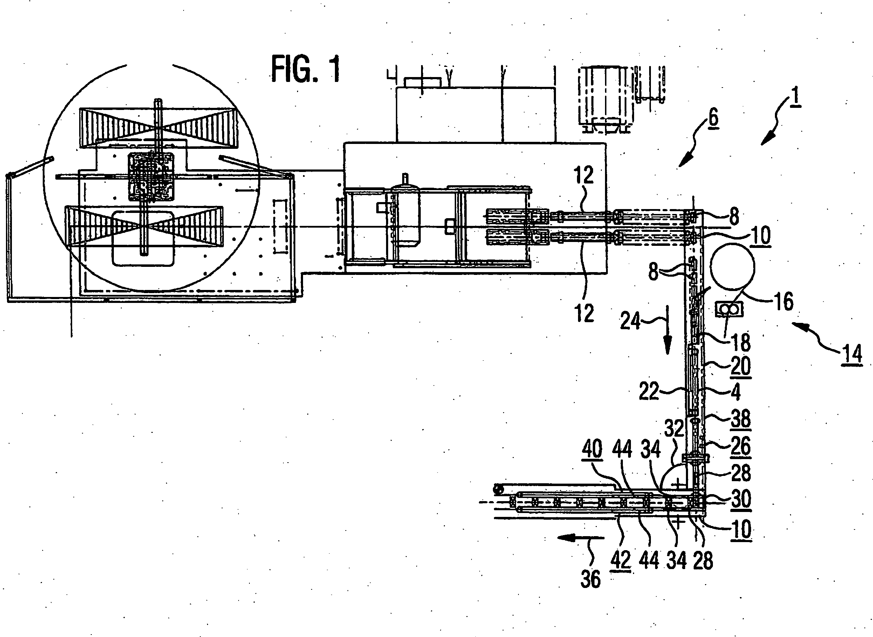

Die Verpackungsanlage 1 gemäß FIG. 1 ist zum Verpacken von Hygieneprodukten in Verpackungsbeutel vorgesehen. Im Ausführungsbeispiel sollen dabei als Hygieneprodukte Papiertaschentücher chargenweise, also als Stapel einzelner Taschentücher, mit einer Chargengröße von jeweils zehn Stück in jeweils einen Verpackungsbeutel eingebracht werden. Alternativ könnten selbstverständlich aber auch andere Chargengößen, beispielsweise die Verpackung von drei oder fünf Papiertaschentüchern in einen Verpackungsbeutel, oder auch andere Hygieneprodukte wie beispielsweise Slipeinlagen, Damenbinden oder dergleichen, vorgesehen sein.

Die Verpackungsanlage 1 ist dabei als so genannter Folienschlauch-Verpacker ausgelegt, bei dem die zu verpackenden Papiertaschentücher chargen- oder stapelweise zunächst in einen Folienschlauch 4 eingeschlagen werden. Dazu umfasst die Verpackungsanlage 1 eine Zuführeinheit 6, über die die Papiertaschentücher in Form von Produktstapeln 8, jeweils umfassend zehn übereinander liegende Papiertaschentücher, einem Transportsystem 10 zugeführt werden. Die Zuführeinheit 6 ist dabei im Ausführungsbeispiel zweibahnig ausgeführt, d. h. in der Art einer parallelen Anordnung zweier Zuführschienen 12 werden die Produktstapel 8 parallel zueinander dem Transportsystem 10 zugeführt. Diese zweibahnige Anordnung ermöglicht insbesondere die Zusammenführung mehrerer Transportstränge für eine gemeinsame Weiterverarbeitung der Produktstapel 8. Damit sind - im Vergleich zur Bereitstellungsrate der Produktstapel 8 bei deren Herstellung - verdoppelte Durchsatzraten beim Verpacken und bei der Weiterverarbeitung der Produktstapel 8 erreichbar.

Dem Transportsystem 10 ist u.a. eine Einschlagstation 14 zugeordnet, in der in einem geeigneten Folienspeicher, beispielsweise in Form von Rollen oder dergleichen die zur Bildung der Verpackungsbeutel vorgesehene Schlauchfolie 16 vorgehalten ist. In der Einschlagstation 14 ist ein geeignet geformter Formschuh 18 vorgesehen, über den die Schlauchfolie 16 um die im Transportsystem 10 transportierten Produktstapel 8 gelegt wird, wobei die Papiertaschentücher in die Folie eingeschlagen werden. Der Formschuh 18 ist dabei derart ausgestaltet, dass die zugeführte Schlauchfolie die durchlaufenden Produktstapel 8 vollständig umgibt, wobei an der Längskante des dabei entstehenden Folienschlauchs 4 eine Überlappung der entsprechenden Kantenbereiche der Folie eingestellt wird.

Die Einschlagstation 14 ist dabei mit geeigneten Mitteln zur örtlichen Synchronisierung der Produktstapel 8 mit der Schlauchfolie 16 versehen. Dabei ist insbesondere vorgesehen, die Produktstapel 8 relativ zur Schlauchfolie 16 korrekt zu positionieren, so dass die Lage der Produktstapel 8 relativ zu einer auf der Schlauchfolie 16 aufgebrachten Bedruckung und/oder relativ zu einer darin eingebrachten Perforation vorgegebene Randbedingungen erfüllt. Damit kann beispielsweise sichergestellt sein, dass eine durch eine Perforation in der Schlauchfolie 16 gebildete Zugriffsöffnung im Verpackungsbeutel auch geeignet relativ zum in den Verpackungsbeutel eingeschlagenen Produktstapel 8 positioniert ist.

Über das Transportsystem 10 sind die zu verpackenden Hygieneprodukte von der Einschlagstation 14 aus gemeinsam mit der sie umgebenden Schlauchfolie 16 einer nachgelagerten ersten Schweißstation 20 zuführbar. Die erste Schweißstation 20 weist dabei in seitlicher Anordnung eine geeignet ausgelegte, insbesondere als Schweißspiegel ausgestaltete Heizeinrichtung 22 auf, über die der Folienschlauch 4 auf eine zum Schweißen ausreichend hohe Temperatur beheizbar ist. Infolge der Beheizung werden die überlappenden Randbereiche der Folie miteinander verschweißt, so dass in der ersten Schweißstation 20 der eigentliche Folienschlauch 4 entsteht. Der Folienschlauch 4 bildet somit eine durchgehende Umhüllung mehrerer aufeinander folgender Produktstapel 8 von Papiertaschentüchern.

Durch den Pfeil 24 ist die Transportrichtung der Produktstapel 8 der Papiertaschentücher auf dem Transportsystem 10 angedeutet. In dieser Transportrichtung gesehen nach der ersten Schweißstation 20 ist eine Vereinzelungs- oder Trenneinheit 26 vorgesehen, über die der mit den Hygieneprodukten versehene Folienschlauch 4 in jeweils einen oder zwei Produktstapel 8 beinhaltende Verpackungsbeutel 28 vereinzelt wird. Diese Vereinzelung ermöglicht nachfolgend eine individualisierte Weiterverarbeitung jeweils einzelner in die Folie eingeschlagener Produktstapel 8.

Nach der Vereinzelung der Produktstapel 8 mündet das Transportsystem 10 in eine Umlenkeinheit 30, in der die mit den Hygieneprodukten versehenen Verpackungsbeutel 28 relativ zur Transportrichtung um einen vorgegebenen Verdrehwinkel 32 gedreht werden. Der Verdrehwinkel 32 ist dabei derart gewählt, dass nach der Drehung ein seitlicher Zugriff auf die noch nicht verschweißten Stirnseiten 34 des den jeweiligen Produktstapel 8 beinhaltenden Verpackungsbeutels 28 möglich ist. Dementsprechend entspricht der Verdrehwinkel 32 basierend auf der Grundfläche des Produktstapels 8 dem von zwei Seiten des Produktstapels 8 aufgespannten Winkel. Im Ausführungsbereich weist der Produkt-stapel 8 eine im Wesentlichen rechteckige Grundfläche auf, so dass ein Verdrehwinkel 32 von 90° gewählt ist.

Im Ausführungsbeispiel ist die Umlenkeinheit 30 dabei derart ausgestaltet, dass im Bereich der Umlenkeinheit 30 - wie durch den Pfeil 36 angedeutet - eine Änderung der Transportrichtung der Hygieneprodükte auf dem Transportsystem 10 um 90° erfolgt. Diese Änderung der Transportrichtung ist dabei vorgesehen, ohne dass eine Änderung der Orientierung der Produktstapel 8 mit dem sie umgebenden Verpackungsbeutel 28 erfolgt, so dass sich relativ zur Transportrichtung gesehen die Orientierung der Produktstapel 8 in der Umlenkeinheit 30 um etwa 90° ändert.

Von der Umlenkeinheit 30 aus sind die die Produktstapel 8 enthaltenden Verpackungsbeutel 28 über das Transportsystem 10 einer nachgeschalteten zweiten Schweißstation 38 zuführbar. In dieser zweiten Schweißstation 38 erfolgt die Verschweißung der Stirnseiten 34 der Verpackungsbeutel 28, wobei auf Grund der Orientierung der Stapel relativ zur Transportrichtung auch hier die Verschweißung von der Seite her erfolgen kann. Dazu umfasst die zweite Schweißstation 38 ebenfalls seitlich angeordnete Heizmittel 40, die beispielsweise als beidseitig zum Produktstrom angeordnete Heizspiegel ausgeführt sein können.

Anschließend mündet das Transportsystem 10 in eine der zweiten Schweißstation 38 nachgeschaltete Kühleinrichtung, in der eine Abkühlung der frisch verschweißten Verpackungsbeutel 28 erfolgt.

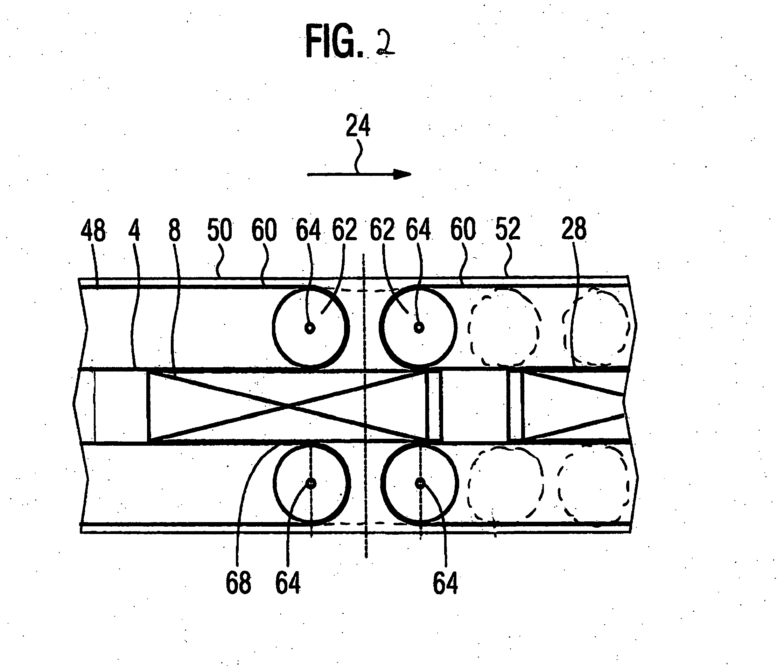

Die Verpackungsanlage 1 ist ebenso wie ihre Komponenten für eine besonders hohe Durchsatzrate beim Verpacken der Produktstapel 8 ausgelegt. Bezüglich der Einschlagstation 14 gemäß FIG. 2 wird diesem Auslegungsziel Rechnung getragen, indem die enge und formschlüssige Umhüllung der Produktstapel mit dem Folienschlauch und der Weitertransport der Produkte durch ein entsprechend gelagertes Transportsystem erfolgt. Für die Umhüllung des Produktstapels 8 mit dem Folienschlauchs 4 ist die Formschülter 18, wie der Formschuh 18 im Stand der Technik ebenfalls bezeichnet wird, als Übergabestation für den eintreffenden Produktstapel 8 von einem ersten, einen Teil des Transportsystems 10 bildenden Fördersystem 50 an ein weiteres, ebenfalls einen Teil des Transportsystems 10 bildendes Fördersystem 52 ausgeführt. In den Fördersystemen 50, 52 wird der zu umhüllende Produktstapel 8 sowie dann auch das mit Folienschlauch 4 umhüllte Produkt unter reibschlüssigen seitlichem Zugriff geführt.

Die Fördersysteme 50, 52, wie dies der ausschnittsweise vergrößerten Darstellung in FIG. 2 entnehmbar ist, sind jeweils als Bandförderer mit seitlich an den Folienschlauch 4 bzw. die Verpackungsbeutel 28 angreifenden Transportbändern 60 ausgeführt. Jedes Fördersystem 50, 52 weist dabei mindestens zwei Einzelbänder 48 auf. In dem hier dargestellten Ausführungsbeispiel weisen die Fördersysteme 50, 52 je zwei Transportbänder 60 im Kopfbereich 66 und jeweils zwei Transportbänder 60 im Fußbereich 68 auf. Somit werden die Produktstapel 8 beim Einführen in die Formschulter 18, im sogenannten Zuführbereich 54, innerhalb der Formschulter 18 im Bereich der Übergabestation 58 sowie auch beim Austritt, dem sogenannten Ausführbereich 56, mit der Umhüllung durch die Schlauchfolie 16 im Kopf- und Fußbereich 66, 68 seitlich von mindestens einem Einzelband 48 gegriffen transportiert. Hinsichtlich der Materialwahl sind die Transportbänder 60 dabei unter anderem für einen guten Reibschluss mit dem Verpackungsbeutel ausgeführt und dabei insbesondere hinsichtlich ihrer Oberfläche geeignet an das Material der Schlauchfolie 16 angepasst. Insbesondere können die Reib-Oberflächen der Transportbänder 60 auch eine geeignete Beschichtung aufweisen.

Im Bereich der als Übergabestation 58 ausgeführten Formschulter 18 sind die Einzelbänder 48 über Umlenkrollen 62 geführt. Auch im Zuführ- und Austragsbereich 54, 56 der Formschulter 18 sind die Einzelbänder 48 über Umlenkrollen 62 geführt. Durch die Anzahl der Einzelbänder 48, hier jeweils zwei pro Fördersystem 50, 52, sind entsprechend, im Zuführ- und Austragsbereich 54, 56 und an der Übergabestation 58 zusammen vier Umlenkrollen 62 vorgesehen. Die Umlenkrollen 62 können dabei als nicht angetriebene, mitlaufende Rollen ausgeführt sein. Für das Auslegungsziel einer zuverlässigen Umhüllung der Produktstapel 8 und dem Ziel die Produktstapel 8 so lange und so weit wie möglich im Eingriff mit den Einzelbänder 48 zu halten, ist die Positionierung der Umlenkrollen 62 zueinander derart gewählt, dass sich im Bereich der Übergabestation 58 keine Lücke bildet. Die Umlenkrollen 62 sind insbesondere in ihrer Dimensionierung und hinsichtlich des Abstandes ihrer Drehachsen 64 voneinander versetzt gelagert. Zwei der vier Umlenkrollen 62, vorzugsweise die im Bereich der Übergabestation 58, können dieselbe Drehachse 64 aufweisen.

Für die überlappende Lagerung der Einzelbänder 48, sind die Umlenkrollen 62 des Kopfbereichs 66 und die des Fußbereichs 68 versetzt positioniert. In der hier gestrichelt dargestellten Ausführung ist das Einzelband 48 des Fördersystems 50 im Kopfbereich 66 verlängert ausgeführt und das Einzelband 48 des Fördersystems 50 im Fußbereich 68 verkürzt ausgeführt. Im Gegenzug dazu ist das Einzelband 48 des Fördersystems 52 im Kopfbereich 66 kürzer als das sich direkt darunter im Fußbereich 68 befindende Transportband 60 des Fördersystems 52. Somit überlagern sich die Transportbänder im Bereich der Übergabestation 58 und gewährleisten, dass sich der Produktstapel 8 vor, während und nach dem Umhüllen mit dem Folienschlauch 4 immer im Fördereingriff durch mindestens eines der Einzelbänder 48 befindet.

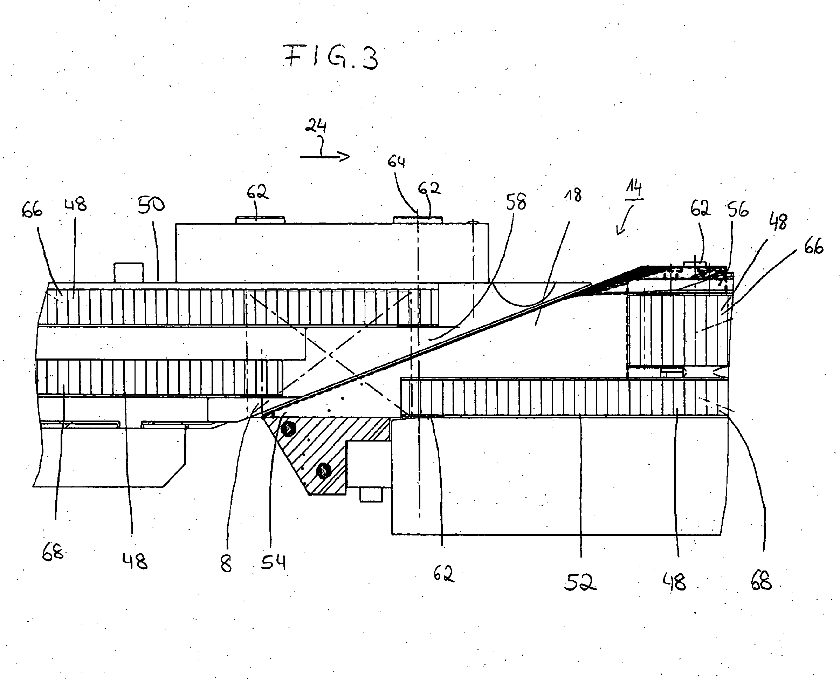

Der in FIG. 3 dargestellte Schnitt durch die Fördersysteme 50, 52 lässt die Positionen der Einzelbänder und der Umlenkrollen gut erkennen. Die Einzelbänder 48 des Fördersystems 50 weisen in diesem Ausführungsbeispiel die gleiche Breite auf und werden voneinander beabstandet geführt. So ist eines der Einzelbänder 48 im Fußbereich 68 und eines im Kopfbereich 66 des Fördersystems 50 positioniert. Dass im Fußbereich 68 geführte Einzelband 48 ist verhältnismässig kürzer als dass im Kopfbereich 66 geführte Einzelband 48 ausgebildet. Diese Ausführungsform ermöglicht es, dass die über versetzt gelagerte Umlenkrollen geführten Einzelbänder 48 im Zuführbereich 54 des Formschuhs 18 den Produktstapel 8 mit reibschlüssigem Zugriff zuverlässig bis in den Bereich der Übergabestation 58 innerhalb des Formschuhs 18 transportieren. So wird der Produktstapel 8 bis zur Übernahme durch eines der Einzelbänder 48 des Fördersystems 52 von mindestens einem der Einzelbänder 48 des Fördersystems 50 geklemmt geführt.

Die vorzugsweise von einer, hier nicht dargestellten, Vorratsrolle abgezogene Schlauchfolie 16 wird in die Einschlagestation 14 geführt, und beginnt im Fußbereich 68 innerhalb der Formschulter 18 den Produktstapel 8 zu umhüllen. Hierbei wird der Produktstapel 8, wie aus FIG. 3 ersichtlich, von den beiden Einzelbändern 48 des Fördersystems 50 geklemmt geführt. Kurz bevor das Einzelband 48 im Fußbereich 68 des Fördersystems 50 den seitlichen Zugriff auf den Produktstapel 8 beendet, hat das Einzelband 48 des Fördersystems 52 bereits begonnen den in diesem Bereich nun schon mit Schlauchfolie 16 versehenen Produktstapel 8 reibschlüssig geklemmt zu führen. Im Bereich der Übergabestation 58 befindet sich der Produktstapel 8, diagonal gesehen, bereits zur Hälfte im Formschuh 18 und ist somit auch zu diesem Teil mit der Schlauchfolie 16 umhüllt. An dieser Position wird der Produktstapel 8 schon zuverlässig von dem sich im Fußbereich 68 des Fördersystems 52 befindenden Einzelbandes 48 geklemmt festgehalten und geführt.

Die überlappende Lagerung des Einzelbandes 48 im Kopfbereich 66 des Fördersystems 50 und des Einzelbandes 48 im Fußbereich 68 des Fördersystems 52 im Bereich der Übergabestation 58 gewährleistet, dass der Produktstapel 8 lückenlos transportiert wird und somit immer mindestens ein Einzelband 48 einen reibschlüssigen Zugriff auf den Produktstapel 8 bzw. den Folienschlauch 4 ausübt. Dies ist insbesondere auch durch die Positionierung der Umlenkrollen 62 dieser Einzelbänder 48, nämlich, wie in diesem Ausführungsbeispiel ersichtlich, derselben Drehachse 64, möglich.

Die Ausgestaltung des Formschuhs 18, vorzugsweise prismenförmig, unterstützt, dass die Schlauchfolie 16 des, vom Einzelband 48 im Fußbereich 68 des Fördersystems 52 weitertransportierten, Produktstapels 8 zuverlässig an den Seitenflächen anliegt.

Im Austragsbereich 56 greift nun zusätzlich ein weiteres sich im Kopfbereich 66 des Fördersystems 52 befindendes Einzelband 48 an den nun entstandenen Folienschlauch 4 an. Sehr gut ersichtlich ist, dass die Einzelbänder 48 des Fördersystems 52 nicht beabstandet, sondern aneinander angrenzend geführt sind. Dadurch sind diese Einzelbänder 48 in ihrer Breite unterschiedlich ausgeführt, was allerdings nicht zwingend erforderlich ist. Für einen zuverlässigen geklemmten Transport können die Einzelbänder 48 des Fördersystems 52 genauso positioniert und ausgeführt sein, wie die Einzelbänder 48 des Fördersystems 50.

Für ein sicheres Einführen der Produktstapel 8 in den Formschuh 18 ist im Kopfbereich 66 ein Niederhalter platziert, der eine Kollision oder gegebenfalls Stau der einfahrenden Produktstapel im Zuführbereich 54 des Formschuh 18 verhindert.

Zum Einstellen für verschiedene Produktstapelgrößen und um Wartungen durchzuführen ist der Formschuh 18 in geteilter Form konstruiert und kann auf einer Linearführung, vorzugsweise zwei, auseinandergezogen werden.

### Bezugszeichenliste

- 1: Verpackungsanlage
- 4: Folienschlauch
- 6: Zuführeinheit
- 8: Produktstapel
- 10: Transportsystem
- 12: Zuführschienen
- 14: Einschlagstätion
- 16: Schlauchfolie
- 18: Formschuh, Formschulter
- 20: erste Schweißstation
- 22: Heizeinrichtung
- 24: Pfeil
- 26: Trenneinheit
- 28: Verpackungsbeutel
- 30: Umlenkeinheit
- 32: Verdrehwinkel
- 34: Stirnseite
- 36: Pfeil
- 38: zweite Schweißstation
- 40: Heizmittel
- 48: Einzelbänder
- 50,52: Fördersystem
- 54: Zuführbereich
- 56: Austragsbereich
- 58: Übergabestation
- 60: Transportband
- 62: Umlenkrolle
- 64: Drehachse
- 66: Kopfbereich
- 68: Fußbereich

## Patentansprüche

1. Verpackungsanlage (1) zum Verpacken von flachen, biegsamen Produkten, insbesondere von Hygieneprodukten, in Verpackungsbeutel (28), mit einer um einen Formschuh (18) kontinuierlich laufenden Schlauchfolie (16) zum Umhüllen der Produkte mit dem gebildeten Folienschlauch (4), einer Fördereinrichtung zum Zu- und Einführen der Produktstapel (8) in den Folienschlauch (4) sowie nachfolgenden Schweißeinrichtungen (20, 38) für die Quer- und Längsschweißung des Folienschlauchs (4), **dadurch gekennzeichnet, dass** die Fördereinrichtung als Bandförderer mit einer Anzahl von jeweils an einer Seite seitlich an den Produkten angreifenden Transportbändern (60) ausgeführt ist, die jeweils aus mindestens zwei Einzelbändern (48) bestehen, und wobei Einzelbänder (48) von in Transportrichtung der Produkte gesehen hintereinander angeordneten Transportbändern (60) an mindestens einer Position der Verpackungsanlage (1) überlappend zueinander angeordnet sind.

2. Verpackungsanlage (1) nach Anspruch 1, **dadurch gekennzeichnet, dass** die jeweils in ihrer Gesamtheit eines der Transportbänder (60) bildenden mindestens zwei Einzelbänder (48) bezogen auf die Transportrichtung der Produkte gesehen nebeneinander und auf derselben Seite des Produktstroms befindlich angeordnet sind.

3. Verpackungsanlage (1) nach Anspruch 1 oder 2, **dadurch gekennzeichnet, dass** die jeweils einem der in Transportrichtung der Produkte gesehen hintereinander angeordneten Transportbänder (60) zugeordneten, überlappend zueinander angeordneten Einzelbänder (48) auf derselben Seite des Produktstroms befindlich angeordnet sind.

4. Verpackungsanlage (1) nach einem der Ansprüche 1 bis 3, **dadurch gekennzeichnet, dass** die Breite der Einzelbänder (48) gleich ist.

5. Verpackungsanlage (1) nach einem der Ansprüche 1 bis 3, **dadurch gekennzeichnet, dass** die Breite der Einzelbänder (48) unterschiedlich ist.

6. Verpackungsanlage (1) nach einem der Ansprüche 1 bis 5, **dadurch gekennzeichnet, dass** die Einzelbänder (48) voneinander beabstandet geführt sind.

7. Verpackungsanlage (1) nach einem der Ansprüche 1 bis 5, **dadurch gekennzeichnet, dass** die Einzelbänder (48) zueinander angrenzend geführt sind.

8. Verpackungsanlage (1) nach einem der Ansprüche 1 bis 6, **dadurch gekennzeichnet, dass** der Abstand der Einzelbänder (48) variabel ist

9. Verpackungsanlage (1) nach einem der Ansprüche 1 bis 8, **dadurch gekennzeichnet, dass** zum ständigen reibschlüssigen seitlichen Zugriff innerhalb des Formschuhs (18) eine Übergabestation (58) vorgesehen ist.

10. Verpackungsanlage (1) nach Anspruch 9, **dadurch gekennzeichnet, dass** die Einzelbänder (48) in einem Zuführ- und Austragsbereich (54, 56) des Formschuhs und an der Übergabestation (58) über Umlenkrollen (62) geführt sind.

11. Verpackungsanlage (1) nach Anspruch 10, **dadurch gekennzeichnet, dass** die Umlenkrollen (62) in ihrer Dimensionierung und/oder hinsichtlich des Abstandes ihrer Drehachsen (64) voneinander in Transportrichtung gesehen versetzt gelagert sind.

12. Verpackungsanlage (1) nach Anspruch 10, **dadurch gekennzeichnet, dass** die Umlenkrollen (62) der überlappend gelagerten Einzelbänder (48) dieselbe Drehachse (64) aufweisen.

13. Verpackungsanlage (1) nach einem der vorherigen Ansprüche, **dadurch gekennzeichnet, dass** der Formschuh (18) in geteilter Form ausgebildet ist.

14. Verpackungsanlage (1) nach Anspruch 13, **dadurch gekennzeichnet, dass** der Formschuh (18) auf einer Linearführung ausgebildet ist.

15. Verpackungsanlage (1) nach einem der Ansprüche 1 bis 14, **dadurch gekennzeichnet, dass** am Formschuh (18) ein Niederhalter vorgesehen ist.

## Claims

1. Packaging facility (1) for packing flat, flexible products, in particular hygiene products, into packaging bags (28), comprising a tubular film (16) which runs continuously around a forming shoe (18) for wrapping the products in the formed film tube (4), a conveyor device for supplying and feeding the product stack (8) into the film tube (4), and downstream welding devices (20, 38) for the transverse and longitudinal welding of the film tube (4), **characterised in that** the conveyor device is a belt conveyor having a number of transport belts (60), one side of each of which laterally acts on the products, the transport belts each consisting of at least two individual belts (48), and individual belts (48) of transport belts (60) which are arranged one behind the other when viewed in the transport direction of the products being arranged so as to overlap one another at at least one position on the packaging facility (1).

2. Packaging facility (1) according to claim 1, **characterised in that** the at least two individual belts (48), which as a whole form one of the transport belts (60), are arranged in each case so as to be side by side and on the same side of the product stream when viewed in relation to the transport direction of the products.

3. Packaging facility (1) according to either claim 1 or claim 2, **characterised in that** the individual belts (48), which are arranged so as to overlap one another and which are each allocated to one of the transport belts (60) which are arranged one behind the other when viewed in the transport direction of the products, are arranged on the same side of the product stream.

4. Packaging facility (1) according to any of claims 1 to 3, **characterised in that** the individual belts (48) have the same width.

5. Packaging facility (1) according to any of claims 1 to 3, **characterised in that** the individual belts (48) have different widths.

6. Packaging facility (1) according to any of claims 1 to 5, **characterised in that** the individual belts (48) are guided at a distance from one another.

7. Packaging facility (1) according to any of claims 1 to 5, **characterised in that** the individual belts (48) are guided adjacently to one another.

8. Packaging facility (1) according to any of claims 1 to 6, **characterised in that** the distance between the individual belts (48) is variable.

9. Packaging facility (1) according to any of claims 1 to 8, **characterised in that** a transfer station (58) is provided inside the forming shoe (18) for providing constant frictional side access.

10. Packaging facility (1) according to claim 9, **characterised in that** the individual belts (48) are guided in a supply and discharge region (54, 56) of the forming shoe and on the transfer station (58) by means of idler pulleys (62).

11. Packaging facility (1) according to claim 10, **characterised in that**, when viewed in the transport direction, the idler pulleys (62) are positioned so as to be offset from one another in terms of the dimensions thereof and/or in relation to the distance between the rotational axes (64) thereof.

12. Packaging facility (1) according to claim 10, **characterised in that** the idler pulleys (62) of the overlapping individual belts (48) have the same rotational axis (64).

13. Packaging facility (1) according to any of the preceding claims, **characterised in that** the forming shoe (18) formed so as to be split.

14. Packaging facility (1) according to claim 13, **characterised in that** the forming shoe (18) is on a linear guide.

15. Packaging facility (1) according to any of claims 1 to 14, **characterised in that** a pressure pad is provided on the forming shoe (18).

## Revendications

1. Installation d'emballage (1) destinée à l'emballage de produits flexibles plats, en particulier de produits d'hygiène, dans des sachets d'emballage (28), avec un film tubulaire (16), tournant de manière continue autour d'un sabot de formage (18) et destiné au conditionnement des produits au moyen du tube de film (4) formé, avec un dispositif de convoyage destiné à l'alimentation et à l'insertion de la pile de produits (8) dans le tube de film (4), ainsi qu'avec des dispositifs de thermoformage (20, 38) pour la soudure transversale et longitudinale du tube de films (4), **caractérisée en ce que** le dispositif de convoyage est exécuté comme un convoyeur à bande avec un certain nombre de bandes de transport (60), agissant respectivement latéralement au niveau des produits, au niveau d'une face, et qui sont composées respectivement d'au moins deux bandes individuelles (48), et dans laquelle les bandes individuelles (48) des bandes de transport (60) disposées les unes derrière les autres, considérées dans la direction de transport des produits, sont disposées en se chevauchant l'une par rapport à l'autre, au niveau d'au moins une position de l'installation d'emballage (1).

2. Installation d'emballage (1) selon la revendication 1, **caractérisée en ce que** les au moins deux bandes individuelles (48) formant respectivement dans leur intégralité l'une des bandes de transport (60), vues par rapport à la direction de transport des produits, sont disposées l'une à côté de l'autre et se situent sur le même côté du flux de produits.

3. Installation d'emballage (1) selon la revendication 1 ou 2, **caractérisée en ce que** les bandes individuelles (48), disposées en se chevauchant l'une par rapport à l'autre, et affectées respectivement à l'une des bandes de transport (60) disposées les unes derrière les autres, considérées dans la direction de transport des produits, se trouvent sur le même côté du flux de produits.

4. Installation d'emballage (1) selon l'une des revendications 1 à 3, **caractérisée en ce que** la largeur des bandes individuelles (48) est identique.

5. Installation d'emballage (1) selon l'une des revendications 1 à 3, **caractérisée en ce que** la largeur des bandes individuelles (48) est différente.

6. Installation d'emballage (1) selon l'une des revendications 1 à 5, **caractérisée en ce que** les bandes individuelles (48) sont guidées en étant espacées les unes des autres.

7. Installation d'emballage (1) selon l'une des revendications 1 à 5, **caractérisée en ce que** les bandes individuelles (48) sont guidées en étant adjacentes les unes aux autres.

8. Installation d'emballage (1) selon l'une des revendications 1 à 6, **caractérisée en ce que** la distance entre les bandes individuelles (48) est variable.

9. Installation d'emballage (1) selon l'une des revendications 1 à 8, **caractérisée en ce qu'**une station de transfert (58) destinée à un accès latéral permanent par friction est prévue à l'intérieur du sabot de formage (18).

10. Installation d'emballage (1) selon la revendication 9, **caractérisée en ce que** les bandes individuelles (48) sont guidées dans une zone d'alimentation et de déchargement (54, 56) du sabot de formage et au niveau de la station de transfert (58), par l'intermédiaire de poulies de renvoi (62).

11. Installation d'emballage (1) selon la revendication 10, **caractérisée en ce que** les poulies de renvoi (62) sont, dans leur dimensionnement et/ou au regard de la distance de leurs axes de rotation (64), mises en place de manière décalée les unes par rapport aux autres, considérées dans la direction de transport.

12. Installation d'emballage (1) selon la revendication 10, **caractérisée en ce que** les poulies de renvoi (62) des bandes individuelles (48) positionnées en se chevauchant présentent le même axe de rotation (64).

13. Installation d'emballage (1) selon l'une des revendications précédentes, **caractérisée en ce que** le sabot de formage (18) est conçu dans une forme divisée.

14. Installation d'emballage (1) selon la revendication 13, **caractérisée en ce que** le sabot de formage (18) est conçu sur un guide linéaire.

15. Installation d'emballage (1) selon l'une des revendications 1 à 14, **caractérisée en ce qu'**un serre-flanc est prévu au niveau du sabot de formage (18).
